# EUROPÄISCHE PATENTSCHRIFT

(11) **EP 1 207 867 B1**
(45) Veröffentlichungstag und Bekanntmachung des Hinweises auf die Patenterteilung: **22.03.2006**
(21) Anmeldenummer: 00954585.6
(22) Anmeldetag: 03.08.2000
(51) Int. Cl.: A61K 31/135, A61K 31/196, A61K 9/52

(54) **RETARDIERTE, ORALE, PHARMAZEUTISCHE DARREICHNUNGSFORMEN MIT TRAMADOL**
ORAL PHARMACEUTICAL FORMS OF ADMINISTRATION WITH A DELAYED ACTION WITH TRAMADOL
FORMES DE PRESENTATION PHARMACEUTIQUES ORALES A ACTION RETARDEE AVEC TRAMADOL

(30) Priorität: 31.08.1999 DE 19940944
(43) Veröffentlichungstag der Anmeldung: 29.05.2002
(60) Teilanmeldung: 05023225.5
(73) Patentinhaber: Grünenthal GmbH, 52078 Aachen (DE)
(72) Erfinder: ZIEGLER, Iris, 52159 Rott-Roetgen (DE); BARTHOLOMÄUS, Johannes, 52080 Aachen (DE)
(86) Internationale Anmeldenummer: PCT/EP2000/007525
(87) Internationale Veröffentlichungsnummer: WO 2001/015681

(56) Entgegenhaltungen:
- EP-A- 0 546 676
- DE-A- 19 927 688

## Beschreibung

Die vorliegende Erfindung betrifft zumindest teilweise retardierte, orale, pharmazeutische Darreichungsformen, deren pharmazeutischer Wirkstoff Tramadol zumindest teilweise als eine in situ gebildete Verbindung mit einer Wasserlöslichkeit ≤ 100 mg/ml vorliegt sowie Verfahren zu deren Herstellung.

Die Applikation pharmakologischer Wirkstoffe in Form retardierter Zubereitungen stellt für viele dieser Wirkstoffe, insbesondere für analgetische Wirkstoffe, eine Therapieverbesserung dar. Die Retardierung ermöglicht es, auch für pharmakologische Wirkstoffe mit einer relativ kurzen Halbwertszeit im Organismus, eine Zubereitung mit lang anhaltender Wirkung zur Verfügung zu stellen und durch gleichmäßigere Blutspiegel außerdem Nebenwirkungen zu vermeiden und die Einhaltung der Dosierungsvorschrift bei den Patienten zu verbessern.

Die Retardierung pharmakologischer Wirkstoffe kann z.B. durch das Einbetten der Wirkstoffe in eine retardierende Matrix oder durch das Aufbringen von retardierenden Filmüberzügen erreicht werden.
Die Retardierung von sehr gut wasserlöslichen Wirkstoffen, wie z.B. Tramadolhydrochlorid, einem Analgetikum zur Bekämpfung starker bis sehr starker Schmerzen, mit Hilfe von Filmüberzügen ist oft aufwendig, da Filmüberzüge für solche Wirkstoffe häufig nur eine unzureichende Diffusionsbarriere darstellen oder sich die Permeabilität dieser Filmüberzüge während der Lagerung ändert (P.B. O'Donnell, J.W. McGinity, "Mechanical Properties of Polymeric Films, Prepared from Aqueous Polymeric Dispersions in Aqueous Polymeric Coatings for Pharmaceutical Dosage Forms", Drugs and the Pharmaceutical Science Vol. 79, ed. J.W. McGinity, Marcel Decker, New York, Basel, Hong Kong 1997).

Die Herstellung von Zubereitungen mit retardierenden Filmüberzügen, die aus wäßriger Dispersion aufgebracht werden, erfordert daher aufwendige Überzugsverfahren mit mehrschichtigen Filmen oder zeitintensive Temperverfahren, wie sie in der US-PS-5,645,858 bzw. in den US-PS 5,580,578, US-PS 5,681,585, US-PS-5,472,712 und in K. Bauer, "Coated Pharmaceutical Dosage Forms", medpharm Scientific Publishers, Stuttgart 1998, B. Sutter, Dissertation, Universität Düsseldorf, 1987 oder in F.N. Christensen, Proceed. Intern. Symp. Contr. Rel. Bioact. Mater. 17, 124, 1990 beschrieben sind.

Die Retardierung pharmazeutischer Wirkstoffe kann auch über die Verminderung ihrer Löslichkeit, z.B. durch die Ausbildung schwer löslicher Salze erfolgen (H. Sucker, Pharmazeutische Technologie, Georg Thieme Verlag, Stuttgart, New York 1991). Der Einsatz solcher schwerlöslichen Salze in Darreichungsformen erfordert jedoch zum Teil sehr aufwendige Herstellungsverfahren für diese Salze.

Aufgabe der vorliegenden Erfindung war es daher, Darreichungsformen zur Verfügung zu stellen, die die Nachteile des Standes der Technik nicht aufweisen.

Überraschenderweise wurde nun gefunden, daß diese Aufgabe durch die Bereitstellung zumindest teilweise retardierter, oraler, pharmazeutischer Darreichungsformen des Tramadols gelöst wird, deren retardierter Anteil des Wirkstoffes als eine in situ gebildete Verbindung aus Tramadol und einem weiteren Wirkstoff und/oder Hilfsstoff mit einer Wasserlöslichkeit ≤ 100 mg/ml vorliegt.

Vorzugsweise beträgt die Wasserlöslichkeit der in situ gebildeten Verbindung ≤ 50 mg/ml, besonders bevorzugt ≤ 30 mg/ml, ganz besonders bevorzugt ≤ 10 mg/ml.

Zur Herstellung der in situ gebildeten Verbindung wird der Wirkstoff Tramadol bevorzugt als wasserlösliches Salz, besonders bevorzugt als Tramadolhydrochlorid, mit einem wasserlöslichen, pharmazeutisch verträglichen Salz eines weiteren aciden pharmazeutischen Wirkstoffes oder Hilfsstoffes umgesetzt, der mit Tramadol eine Verbindung mit einer Wasserlöslichkeit ≤ 100 mg/ml, bevorzugt ≤ 50 mg/ml, besonders bevorzugt ≤ 30 mg/ml und ganz besonders bevorzugt ≤ 10 mg/ml bildet. Diese Verbindungen werden als in Wasser schwer lösliche Verbindungen klassifiziert.

In situ Bildung im Sinne der Erfindung bedeutet, daß das Tramadot bzw. dessen wasserlösliches Salz, vorzugsweise bei der Herstellung der erfindungsgemäßen Darreichungsformen, mit einem aciden, weiteren pharmazeutischen Wirkstoff oder Hilfsstoff bzw. deren jeweiligen wasserlöslichen Salzen gemischt, mehrfach angefeuchtet, gegebenenfalls extrudiert oder unter sonstigem Energieeintrag formuliert wird.

Als wasserlösliches Salz des aciden, weiteren pharmazeutischen Wirkstoffes und/oder biologisch verträglichen Hilfsstoffes zur Herstellung der in situ gebildeten Tramadol-Verbindung wird vorzugsweise das Natriumsalz von Diclofenac, Naproxen, der Acetylsalicylsäure, der Salicylsäure, der Benzoesäure, oder des Saccharins, Cyclamats oder Acesulfams eingesetzt.

Die erfindungsgemäßen retardierten, oralen, pharmazeutischen Darreichungsformen können die Tramadolkomponente und den weiteren pharmazeutischen Wirk- und/oder Hilfsstoff in beliebigen molaren Verhältnissen enthalten.

In einer bevorzugten Ausführungsform der erfindungsgemäßen Darreichungsformen liegt die Tramadolkomponente im Überschuß vor und wird mit wenigstens zwei unterschiedlichen Geschwindigkeiten freigesetzt. Dies bedeutet, daß neben der retardierten Freisetzung des Tramadols aus der in situ gebildeten Verbindung ein Teil des Wirkstoffes schnell als Initialdosis freigesetzt wird.

In einer weiteren bevorzugten Ausführungsform der erfindungsgemäßen Darreichungsformen liegen die Tramadolkomponente und der acide, weitere pharmazeutische Wirkstoff oder Hilfsstoff in äquimolaren Mengen als die in situ gebildete, schwer lösliche Verbindung vor. Damit wird erreicht, daß die beiden Wirkstoffe bzw. Wirkstoff/Hilfsstoff trotz ihrer ursprünglich ggf. unterschiedlichen Wasserlöslichkeit mit derselben Geschwindigkeit retardiert freigesetzt werden.

In einer besonders bevorzugten Ausführungsfom der erfindungsgemäßen Darreichungsformen sind Tramadolhydrochlorid und Diclofenac-Natrium in-situ zu einer sehr schwer löslichen Verbindung mit einer Wasserlöslichkeit von ≤ 0,3 mg/ml umgesetzt worden. Vorzugsweise beträgt das Mengenverhältnis von Tramadol zu Diclofenac in diesen erfindungsgemäßen Darreichungsformen 0,5:1 bis 4:1, besonders bevorzugt 1:1 bis 2:1. Vorzugsweise wird Tramadol im Überschuß zur in situ Umsetzung mit Diclofenac eingesetzt, so daß diese Darreichungsformen eine schnell freisetzende Initialdosis von Tramadol und eine retardierte Freisetzung von Tramadol und Diclofenac mit derselben Geschwindigkeit besitzen. Durch die Kombination mit dem sofort freigesetzten Wirkstoff als Initialdosis läßt sich eine schnelle Schmerzlinderung erzielen. Die langsame Freisetzung der Wirkstoffe aus der retardierten Form ermöglicht dann die Aufrechterhaltung der analgetischen Wirkung über längere Zeit.

Ebenso bevorzugte retardierte erfindungsgemäße Darreichungsformen enthalten eine in situ gebildete Verbindung, die aus äquimolaren Mengen der Wirkstoffe Tramadol und Diclofenac gebildet wurde, so daß die Gesamtmenge jedes Wirkstoffes retardiert mit derselben Geschwindigkeit freigesetzt wird. Die erfindungsgemäßen, zumindest teilweise retardierten, oralen,
pharmazeutischen Darreichungsformen des Tramadols liegen vorzugsweise multipartikulär, besonders bevorzugt als Granulat, Mikropartikel, Mikrotabletten oder Pellets, ganz besonders bevorzugt als Pellets, und gegebenenfalls in Kapseln abgefüllt, formuliert vor. Die Pellets sind bevorzugt durch Extrusion
und Spheronisation hergestellte Pellets, die vorzugsweise einen Durchmesser von 0,1 bis 3 mm aufweisen.
Die erfindungsgemäßen Darreichungsformen können auch als Dragees oder Tabletten, vorzugsweise schnell zerfallende Tabletten, formuliert vorliegen. Die Tabletten können aus verpreßten Pellets bestehen, die besonders bevorzugt schnell zerfallen.

Ein besonderer Vorteil der erfindungsgemäßen Darreichungsformen besteht darin, daß eine Retardierung des Tramadols bereits bei der Herstellung der Darreichungsform durch die in situ Bildung einer Verbindung mit einer Wasserlöslichkeit ≤ 100 mg/ml aus Tramadol und einem weiteren Wirkstoff und/oder Hilfsstoff ohne den Einsatz einer retardierenden Matrix und/oder eines retardierenden Überzuges erreicht wird.

Die erfindungsgemäßen pharmazeutischen Darreichungsformen weisen vorzugsweise wenigstens einen magensaftresistenten Überzug auf, der sich pH-abhängig auflöst. Durch diesen Überzug wird erreicht, daß sie den Magentrakt unaufgelöst passieren und der/die Wirkstoff(e) und/oder Hilfstoff(e) erst im Darmtrakt zur kontrollierten Freisetzung gelangt/gelangen. Der magensaftresistente Überzug kann aus wäßriger Lösung oder Dispersion und/oder aus organischer Lösung aufgebracht werden. Vorzugsweise löst sich der magensaftresistente Überzug bei einem pH-Wert zwischen 5 und 7 auf. Der magensaftresistente Überzug besteht vorzugsweise aus Schellack, Polymethacrylsäureethylacrylat- oder Methacrylsäure/Methylacrylat/Methylmethacrylat-Copolymer, Methacrylsäure/Methylmethacrylat Copolymeren, Hydroxypropylmethylcelluloseacetatsuccinat, Celluloseacetatphthalat, Polyvinylacetatphthalat, Hydroxypropylmethylcellulosephthalat und/oder Celluloseacetattrimellitat.

Eine über die in situ verursachte Retardierung hinausgehende Retardierung und somit auch eine weitere Modifizierung der Freisetzung des Wirkstoffes Tramadol und gegebenenfalls weiterer Wirkstoffe kann nach verschiedenen dem Fachmann bekannten Methoden erfolgen.

Vorzugsweise kann eine weitere Retardierung mit Hilfe von retardierenden Überzügen erreicht werden. Geeignete, retardierende Überzüge umfassen wasserunlösliche Wachse oder Polymere, wie z.B. Acrylharze, vorzugsweise Poly(meth)acrylate, oder wasserunlösliche Cellulosen, vorzugsweise Ethylcellulose. Diese Materialien sind aus dem Stande der Technik, z.B. Bauer, Lehmann, Osterwald, Rothgang "Uberzogene Arzneiformen", Wissenschaftliche Verlagsgesellschaft mbH Stuttgart, 1988, Seite 69 ff., bekannt.

Neben den wasserunlösüchen Polymeren können zur Einstellung der Freisetzungsgeschwindigkeit der Wirkstoffe die Retardüberzüge gegebenenfalls auch nicht retardierende, vorzugsweise wasserlösliche Polymere in Mengen bis zu 30 Gew.%, wie Polyvinylpyrrolidon oder wasserlösliche Cellulosen, vorzugsweise Hydroxypropylmethylcellulose oder Hydroxypropylcellulose, und/oder hydrophile Porenbildner, wie Saccharose, Natriumchlorid oder Mannitol und/oder die bekannten Weichmacher enthalten.

Ebenfalls bevorzugt können die erfindungsgemäßen Darreichungsformen zur weiteren Retardierung die schwer lösliche in situ gebildete Tramadolverbindung auch in einer retardierenden Matrix, vorzugsweise gleichmäßig verteilt, enthalten.

Als Matrixmaterialien können physiologisch verträgliche, hydrophile Materialien verwendet werden, welche dem Fachmann bekannt sind. Vorzugsweise werden als hydrophile Matrixmaterialien Polymere, besonders bevorzugt Celluloseether, Celluloseester und/oder Acrylharze verwendet. Ganz besonders bevorzugt werden als Matrixmaterialien Ethylcellulose, Hydroxypropylmethylcellulose, Hydroxypropylcellulose, Hydroxymethylcellulose, Poly(meth)acrylsäure und/oder deren Derivate, wie deren Salze, Amide oder Ester eingesetzt.

Ebenfalls bevorzugt sind Matrixmaterialien aus hydrophoben Materialien, wie hydrophobe Polymere, Wachse, Fette, langkettigen Fettsäuren, Fettalkohole oder entsprechenden Ester oder Ether oder deren Gemische. Besonders bevorzugt werden als hydrophobe Materialien Mono- oder Diglyceride von C₁₂₋C₃₀-Fettsäuren und/oder C₁₂-C₃₀-Fettalkohole und/oder Wachse oder deren Gemische eingesetzt.

Es ist auch möglich, Mischungen der genannten hydrophilen und hydrophoben Materialien als retardierendes Matrixmaterial einzusetzen. Bevorzugt wird die Freisetzung der Wirkstoffe so einzustellen sein, daß die erfindungsgemäßen Darreichungsformen höchstens zweimal, besonders bevorzugt nur einmal täglich verabreicht werden müssen. Dem Fachmann ist aufgrund der Wirkung der Analgetika bekannt, in welchen Dosierungen diese einzusetzen sind, damit die gewünschte Wirkung erreicht wird.

Eine zusätzliche Möglichkeit, die Freisetzung des Wirkstoffes Tramadol und gegebenenfalls weiterer Wirkstoffe aus den erfindungsgemäßen Darreichungsformen zu modifizieren, ist durch die Variation ihrer Oberfläche und/oder durch den Einsatz hydrophiler Hilfsstoffe möglich. Eine Vergrößerung der Oberfläche, z.B. durch den Einsatz kleinerer Pellets, bewirkt eine schnellere Freisetzung der Wirkstoffe. Eine Erhöhung der Menge hydrophiler Hilfsstoffe im Pelletkem, wie z.B. Lactose, führt ebenfalls zu einer schnelleren Freisetzung des Wirkstoffes/der Wirkstoffe.

Die erfindungsgemäßen Darreichungsformen eignen sich bevorzugt zur Bekämpfung von Schmerzen oder zur Behandlung von Haminkontinenz, Husten, inflammatorischen und/oder allergischen Reaktionen, Depressionen, Drogen und/oder Alkoholmißbrauch, Gastritis, Diarrhoe, cardiovaskulären Erkrankungen, Atemwegserkrankungen, seelischen Erkrankungen oder Epilepsie.

Ein weiterer Gegenstand der Erfindung sind Verfahren zur Herstellung der erfindungsgemäßen, zumindest teilweise retardierten, oralen, pharmazeutischen Darreichungsformen, in denen Tramadol bzw. dessen Salz und ein acider, weiterer pharmazeutischer Wirkstoff oder Hilfsstoff bzw. deren jeweilige wasserlöslichen Salze und gegebenenfalls weitere Hilfsstoffe gemischt, mehrfach angefeuchtet und unter Energieeintrag formuliert werden. Die Mischung wird bevorzugt mit wässrigen Medien, besonders bevorzugt mit Wasser oder wässrigen Bindemittellösungen jeweils angefeuchtet. Der Energieeintrag erfolgt vorzugsweise in Form von Druck und/oder Wärme.

In einer bevorzugten Ausführungsform des erfindungsgemäßen Verfahrens wird die Mischung einem mehrmaligem Anfeuchten und granulieren und wenigstens einer Extrusion unterworfen und dann gegebenenfalls endformuliert.

Vorzugsweise erfolgt nach jedem Befeuchtungs- und Granulierungsschritt eine Extrusion und/oder Trocknung der Mischung.

Vorzugsweise wird die Mischung nach dem mehrfachen Anfeuchten, Granulieren, Extrudieren und/oder Trocknen pelletisiert und ggf. nach Abmischen mit weiteren Hilfsstoffen zu Tabletten verpreßt. Vorzugsweise erfolgt vor der Pelletisierung eine Extrusion.

In einer besonders bevorzugten Ausführungsform des erfindungsgemäßen Verfahrens wird die angefeuchtete Mischung der Salze granuliert, extrudiert, erneut angefeuchtet und granuliert, extrudiert und anschließend gerundet. In einem weiteren besonders bevorzugten Verfahren wird die angefeuchtete Mischung granuliert, getrocknet, erneut angefeuchtet und granuliert, extrudiert und anschließend gerundet.

Die Pellets werden vorzugsweise vor dem Verpressen mit einem magensaftresistenten Überzug versehen.

In einer besonders bevorzugten Ausführungsform des erfindungsgemäßen Verfahrens werden zur Herstellung der in-situ gebildeten Verbindung Tramadolhydrochlorid und Diclofenac-Natrium eingesetzt.

Sofern eine Endformulierung der erfindungsgemäßen Darreichungsformen erfolgt, kann diese nach den verschiedenen dem Fachmann bekannten Methoden erfolgen.

Die erfindungsgemäßen Darreichungsformen können außerdem je nach Ausführungsform als weitere Bestandteile die üblichen, dem Fachmann bekannten Hilfs- und Zusatzstoffe enthalten. Sofern die erfindungsgemäßen Darreichungsformen Überzüge aufweisen, können diese nach üblichen Verfahren, wie z.B. Dragieren, Aufsprühen von Lösungen, Dispersionen oder Suspensionen, durch Schmelzverfahren oder durch Pulverauftragsverfahren aufgebracht werden.

Die erfindungsgemäßen Darreichungsformen zeichnen sich überraschenderweise dadurch aus, daß die Freisetzung des Wirkstoffes Tramadol und gegebenfalls weiterer Wirkstoffe aus den erfindungsgemäßen Darreichungsformen durch eine Variation der Freisetzungsbedingungen im üblichen Rahmen, wie zum Beispiel durch die lonenkonzentration der Puffer, Anwesenheit oberflächenaktiver Substanzen, Einsatz verschiedener Puffertypen und/oder unterschiedlicher mechanischer Belastung nicht beeinflußt wird. Auch nach längerer Lagerung bei erhöhter Temperatur bis 40 °C verändern sich die Geschwindigkeiten für die Freisetzung der/des Wirkstoffe(s) aus den erfindungsgemäßen Darreichungsformen nicht. Die erhaltenen Darreichungsformen zeigen ein lagerstabiles Freisetzungsprofil.

Diese Freisetzung der Wirkstoffe aus den erfindungsgemäßen, zumindest teilweise retardierten, oralen, pharmazeutischen Darreichungsformen erfolgt nach einer Freisetzungskinetik, die sonst nur durch aufwendige Matrixsysteme erreicht werden kann. Überraschenderweise zeigt sich, daß eine Retardierung der pharmazeutischen Wirkstoffe ohne den Einsatz üblicher Retardierungssysteme möglich ist. Dabei können die Freisetzungsprofile durch Variation der Größe der Arzneiform und die Inkorporation löslicher Hilfsstoffe unter Beibehaltung der gemeinsamen Freisetzungsgeschwindigkeit für beide Wirkstoffe modelliert werden. Diese Freisetzung beider Wirkstoffe aus den in situ hergestellten schwerlöslichen Tramadol-Diclofenac-Verbindungen erfolgt überraschenderweise trotz ihrer sehr kleinen Partikelgröße von durchschnittlich ≤ 5 µm genau so retardiert wie die Freisetzung von separat hergestellten Salzen aus Tramadol und Diclofenac aus identischen Darreichungsformen nur mit wesentlich größeren Partikelgrößen von ca. 20 - 100 µm.

Da die Retardierung des Wirkstoffes Tramadol und gegebenenfalls weiterer Wirkstoffe in den erfindungsgemäßen Darreichungsformen ohne den Einsatz weiterer Retardierungssysteme erreicht werden kann, lassen sich diese zeitsparender und kostengünstiger mit ausgezeichneter Reproduzierbarkeit herstellen.

Die Löslichkeit der Verbindungen aus Tramadol und dem entsprechenden weiteren aciden pharmazeutischen Wirkstoff oder Hilfsstoff wurde wie folgt bestimmt:

Von den jeweiligen nach dem erfindungsgemäßen Verfahren hergestellten, nicht mit einem retardierenden Überzug versehenen Pellets enthaltend eine Verbindung aus Tramadol, (1RS,2RS)-2-[(Dimethylamino)methyl]-1-(3-methoxyphenyl)-cyclohexanol, und dem entsprechenden aciden, pharmazeutischen Wirkstoff oder Hilfsstoff wurde bei 25 °C eine so große Menge in ionenfreies Wasser gegeben (entsprechend ca. 25 mg Pellets auf 5 ml ionenfreies Wasser), daß eine bei dieser Temperatur gesättigte Lösung entstand, die auch nach 24 Stunden schütteln (Vibrax, Schüttelbadeinstellung = 1200 bei 25 °C) noch gesättigt war.
Der entsprechende Sättigungslöslichkeitsbereich wurde in Vorversuchen anhand des jeweiligen separat hergestellten Salzes aus Tramadol und dem entsprechenden aciden pharmazeutischen Wirkstoff oder Hilfsstoff abgeschätzt.

Nach dem Absetzen der gesättigten Lösung wurde der klare Überstand abpipettiert und bei 3500 Umdrehungen pro Minute für 5 Minuten zentrifugiert. Ein Teil des so erhaltenen klaren Überstandes wird in ein HPLC-Probenfläschen überführt und die Konzentration der Verbindung aus Tramadol und dem entsprechenden aciden pharmazeutischen Wirkstoff oder Hilfsstoff wird gegen Tramadolhydrochlorid als Standard bestimmt.

Das Freisetzungsprofil der in den Beispielen hergestellten Präparate wurde wie folgt bestimmt:

Die Zubereitungen wurden entweder in einem Drehkörbchenapparat (Beispiele 1 bis 6) oder in einer Blattrührapparatur (Beispiel 7) gemäß Europäischem Arzneibuch bei einer Temperatur von 37°C (± 0,5 °C) und einer Umdrehungsgeschwindigkeit von 100 min⁻¹ bzw. 50 min⁻¹ geprüft. In Beispiel 1 wurde die Zubereitung zehn Stunden, in Beispiel 6 fünf Stunden und in Beispiel 5 vier Stunden in 900 ml künstlichem Darmsaft ohne Enzyme (pH 7,2) geprüft. In den Beispielen 2 bis 4 und 7 wurde die Zubereitung zunächst zwei Stunden lang in 600 ml künstlichem Magensaft ohne Enzyme (pH 1,2) und anschließend weitere acht Stunden lang in 900 ml künstlichem Darmsaft ohne Enzyme (pH 7,2) getestet.
Die zum jeweiligen Zeitpunkt freigesetzten Menge der Wirkstoffe wurde durch HPLC bestimmt. Die dargestellten Werte und Kurven sind die Mittelwerte aus jeweils 3 Proben.

Im folgenden wird die Erfindung anhand der Beispiele erläutert. Die Erläuterungen sind lediglich beispielhaft und schränken den allgemeinen Erfindungsgedanken nicht ein.

### Beispiele:

### Beispiel 1:

125 g Tramadolhydrochlorid, 125 g Diclofenac-Natrium und 250 g mikrokristalline Cellulose (Avicel PH 101, FMC) wurden in einem Kenwood Chef Mixer 10 Minuten homogen gemischt und anschließend mit einer zum Anfeuchten ausreichenden Menge Wasser granuliert. Die klebrige, klumpige Granulatmasse wurde anschließend in einem Nica Extruder (Typ E140) mit 1,0 mm Extrusionsmatrize extrudiert. Während die Extrudatstränge anfänglich noch extrem klebrig waren, erfolgte im Verlauf der Extrusion bereits die Umsetzung zu einem sehr trockenen Extrudat mit unzureichender Plastizität für die anschließende Spheronisation. Das Extrudat wurde erneut angefeuchtet und erneut granuliert. Das resultierende Granulat wurde erneut im Nica Extruder extrudiert und das feuchte Extrudat anschließend mit einem Nica Spheroniser (Typ S450) zu gleichmäßig großen, runden Pellets ausgerundet. Die Pellets wurden im Trockenschrank bei einer Temperatur von ca. 50°C getrocknet und in Siebfraktionen fraktioniert, wobei ≥ 90% der Pellets innerhalb der gewünschten Siebklasse von 800 - 1250 µm lagen.

| Zusammensetzung der Pellets: | |
|---|---|
| Tramadol-HCl | 50 mg |
| Diclofenac-Na | 50 mg |
| Mikrokristalline Cellulose (Avicel PH 101, FMC) | 100 mg |
| | 200 mg |

Die Wasserlöslichkeit des Wirkstoffes Tramadol aus der in situ gebildeten Verbindung wurde für die vorstehend hergestellten Pellets nach der oben angegebenen Methode zu 0,36 mg/ml bestimmt.

Das Freisetzungsprofil, bestimmt nach der oben angegebenen Methode, war wie folgt:

| Zeit | Freigesetzte Menge in | |
|---|---|---|
| in min | mg aus 200 mg Pellets | |
| | für Tramadol | für Diclofenac |
| 30 | 10 | 7 |
| 120 | 18 | 15 |
| 300 | 26 | 24 |
| 600 | 35 | 33 |

### Beispiel 2:

200 g Trämadolhydrochlorid, 100 g Diclofenac-Natrium, 22 g gepulverte Bemsteinsäure und 332 g mikrokristalline Cellulose (Avicel PH 101, FMC) wurden in einem Kenwood Chef Mixer 10 Minuten homogen gemischt und analog Beispiel 1 zu Pellets verarbeitet.

| Zusammensetzung der Pellets: | |
|---|---|
| Tramadolhydrochlorid | 100 mg |
| Diclofenac-Natrium | 50 mg |
| Bernsteinsäure, gepulvert | 11 mg |
| Mikrokristalline Cellulose (Avicel PH 101, FMC) | 166 mg |
| | 327 mg |

Anschließend wurden 500 g der klassierten Pellets in der Wirbelschicht bei einer Zulufttemperatur von 40°C mit einer wäßrigen Schellacklösung bei einem Gewichtsauftrag von 5 Gew.% Schellack, bezogen auf das Gewicht der Pellets, magensaftresistent überzogen.

| Filmüberzug für 500 g Pellets: | |
|---|---|
| wäßrige Schellacklösung ASL 125 | |
| (20% Feststoffgehalt, Marchand & Cie) | 125 g |
| Triethylcitrat | 1,25 g |
| Wasser | 136,25 g |

Das Freisetzungsprofil, bestimmt wie vorstehend angegeben, war wie folgt:

| Zeit | Freigesetzte Menge in | |
|---|---|---|
| in min | mg aus 344 mg Pellets | |
| | für Tramadol | für Diclofenac |
| 120 | 0 | 0 |
| 240 | 61 | 10 |
| 480 | 76 | 25 |
| 600 | 84 | 28 |

### Beispiel 3:

1,25 kg Tramadolhydrochlorid, 1,25 kg Diclofenac-Natrium, 1,0 kg Lactose Monohydrat, 0,75 kg mikrokristalline Cellulose (Avicel PH 101, FMC) und 0,75 kg kolloidale mikrokristalline Cellulose (Avicel RC 591, FMC) wurden in einem Diosna (Typ P25) gemischt und granuliert. Die Herstellung der Pellets erfolgte analog zu Beispiel 1 mit nachfolgenden Änderungen. Das klebrige, feuchte Granulat wurde nach der Granulation nicht extrudiert, sondern direkt auf mit Folie verschlossenen Hordenblechen ausgebreitet und für 20 Minuten im Trockenschrank bei 50 bis 70°C erhitzt, wobei Feuchtigkeitsverluste vermieden wurden. Danach wurde das Granulat erneut angefeuchtet und granuliert. Die Extrusion erfolgt im Nica Typ (E140 Extruder) mit 0.8 mm Extrusionseinsatz. Die Spheronisation des Extrudates erfolgte im Nica Spheroniser (Typ S450). Nach der Trocknung der Pellets im Trockenschrank wurden diese klassiert, wobei zwischen ≥ 90 % der Pellets in der gewünschten Siebklasse zwischen 0.63 und 1.0 mm lagen

| Zusammensetzung der Pellets: | |
|---|---|
| Tramadolhydrochlorid | 75 mg |
| Diclofenac-Natrium | 75 mg |
| Lactose Monohydrat | 60 mg |
| Mikrokristalline Cellulose (Avicel PH 101, FMC) | 45 mg |
| Kolloidale Mikrokristalline Cellulose (Avicel RC 591, FMC) | 45 mg |
| | 300 mg |

5 kg der Pellets wurden dann im Hüttlin Kugelcoater bei einer Zulufttemperatur von 40°C mit 21 % Gewichtsprozent Eudragit L-55, bezogen auf das Gesamtgewicht der Pellets, aus wäßriger Dispersion der nachstehenden Zusammensetzung überzogen.

| Filmüberzug für 5 kg Pellets: | |
|---|---|
| Eudragit L30D-55 (Röhm, 30% ige, wäßrige Dispersion von Polymethacrylsäureethylacrylat 1:1 Copolymer) | 3500 g |
| Eudragit NE30D (Röhm, 30% ige, wäßrige Dispersion von Polyethylacrylatmethylmethacrylat Copolymer) | 315 g |
| Triethylcitrat | 175 g |
| Talkum, mikronisiert | 262,5 g |
| Wasser | 3657,5 g |

### Zusammensetzung der Kapseln:

Jeweils 400 mg der überzogenen Pellets wurden zusammen mit 46 mg Tramadolinitialdosispellets (entsprechend 25 mg Tramadolhydrochlorid, 10,5 mg Avicel PH 105 und 10,5 mg I-HPC LH31) auf einer Zanasi E6 Steckkapselmaschine mit 2 Pelletdosierstationen in Hartgelatinekapseln der Größe 0 gefüllt.

Das Freisetzungsprofil, bestimmt wie vorstehend angegeben, war wie folgt:

| Zeit | Freigesetzte Menge in mg | |
|---|---|---|
| in min | pro Kapsel | |
| | für Tramadol | für Diclofenac |
| | (Dosis 100 mg) | (Dosis 75 mg) |
| 30 | 25 | 0 |
| 120 | 28 | 0 |
| 240 | 56 | 29 |
| 480 | 79 | 50 |
| 600 | 85 | 56 |

### Beispiel 4:

1,5 kg Tramadolhydrochlorid, 1,0 kg Diclofenac-Natrium, 1,0 kg Lactose Monohydrat, 0,75 kg mikrokristalline Cellulose (Avicel PH 101, FMC) und 0,75 kg kolloidale mikrokristalline Cellulose (Avicel RC 591, FMC) wurden in einem Diosna (Typ P25) gemischt und granuliert. Die Herstellung der Pellets erfolgte analog zu Beispiel 3 mit nachfolgenden Änderungen. Die Umsetzung des Diclofenac-Natrium mit Tramadolhydrochlorid erfolgte direkt nach der ersten Granulation im Mischer durch Aufheizen des Doppelmantels auf eine Temperatur von 70°C für einen Zeitraum von 30 min, wobei der Rührflügel einige Male kurzzeitig aktiviert wurde. Nach der Umsetzung erfolgte die zweite Granulation direkt ohne Entleerung.

| Zusammensetzung der Pellets: | |
|---|---|
| Tramadolhydrochlorid | 75 mg |
| Diclofenac-Natrium | 50 mg |
| Lactose Monohydrat | 50 mg |
| Mikrokristalline Cellulose (Avicel PH 101, FMC) | 37,5 mg |
| Kolloidale Mikrokristalline Cellulose (Avicel RC 591, FMC) | 37,5 mg |
| | 250 mg |

5 kg Pellets wurden dann im Hüttlin Kugelcoater bei einer Zulufttemperatur von 40°C mit 22 Gew.-% Eudragit L-55, bezogen auf das Gesamtgewicht der Pellets, aus wäßriger Dispersion mit folgender Zusammensetzung überzogen.

### Filmüberzug für 5 kg Pellets:

| | |
|---|---|
| Eudragit L30D-55 (Röhm, | 3667 g |
| 30% ige, wäßrige Dispersion von | |
| Polymethacrylsäureethylacrylat 1:1 Copolymer) | |
| Triethylcitrat | 220 g |
| Talkum, mikronisiert | 550 g |
| Wasser | 4913,5 g |

### Zusammensetzung der Kapseln:

Jeweils 348 mg der überzogenen Pellets wurden zusammen mit 46 mg Tramadolinitialdosispellets (entsprechend 25 mg Tramadolhydrochlorid, 10,5 mg Avicel PH 105 und 10,5 mg I-HPC LH31) auf einer Zanasi E6 Steckkapselmaschine mit 2 Pelletdosierstationen in Hartgelatinekapseln der Größe 0 gefüllt.

Das Freisetzungsprofil, bestimmt wie vorstehend angegeben, war wie folgt:

| Zeit | Freigesetzte Menge in | |
|---|---|---|
| in min | mg pro Kapsel | |
| | für Tramadol | für Diclofenac |
| | Dosis (100 mg) | Dosis (50 mg) |
| 30 | 27 | 0 |
| 120 | 32 | 0 |
| 240 | 78 | 24 |
| 480 | 94 | 40 |
| 600 | 99 | 45 |

### Beispiel 5:

100 g Tramadolhydrochlorid, 69 g Saccharin-Natrium und 169 g mikrokristalline Cellulose (Avicel PH 101, FMC) wurden in einem Kenwood Chef Mixer 10 Minuten homogen gemischt und anschließend analog Beispiel 1 zu Pellets verarbeitet.

| Zusammensetzung der Pellets: | |
|---|---|
| Tramadolhydrochlorid | 100 mg |
| Saccharin-Natrium | 69 mg |
| Mikrokristalline Cellulose (Avicel PH 101, FMC) | 169 mg |
| | 338 mg |

Das Freisetzungsprofil, bestimmt wie vorstehend angegeben, war wie folgt:

| Zeit in min | Freigesetzter Anteil in % für Tramadol |
|---|---|
| 30 | 84 |
| 120 | 100 |
| 240 | 104 |

### Beispiel 6:

100 g Tramadolhydrochlorid, 84 g Naproxen-Natrium und 184 g mikrokristalline Cellulose (Avicel PH 101, FMC) wurden in einem Kenwood Chef Mixer 10 Minuten homogen gemischt und anschließend analog Beispiel 1 zu Pellets verarbeitet.

| Zusammensetzung der Pellets: | |
|---|---|
| Tramadolhydrochlorid | 100 mg |
| Naproxen-Natrium | 84 mg |
| Mikrokristalline Cellulose (Avicel PH 101, FMC) | 184 mg |
| | 368 mg |

Das Freisetzungsprofil, wie vorstehend angegeben bestimmt, war wie folgt:

| Zeit in min | Freigesetzter Anteil in % | |
|---|---|---|
| | für Tramadol | für Naproxen |
| 30 | 72 | 55 |
| 120 | 91 | 88 |
| 240 | 101 | 100 |
| 300 | 102 | 102 |

### Beispiel 7:

1,5 kg Tramadolhydrochlorid, 1,0 kg Diclofenac-Natrium, 1,0 kg Lactose Monohydrat, 0,75 kg mikrokristalline Cellulose (Avicel PH 101, FMC) und 0,75 kg kolloidale mikrokristalline Cellulose (Avicel RC 591, FMC) wurden in einem Diosna (Typ P 25) 10 Minuten homogen gemischt und analog Beispiel 3 zu Pellets verarbeitet.

| Zusammensetzung der Pellets | |
|---|---|
| Tramadolhydrochlorid | 75 mg |
| Diclofenac-Natrium | 50 mg |
| Lactose Monohydrat | 50 mg |
| Mikrokristalline Cellulose (Avicel PH 101, FMC) | 37,5 mg |
| Kolloidale Mikrokristalline Cellulose (Avicel RC 591, FMC) | 37,5 mg |
| | 250 mg |

Anschließend wurden 5 kg der Pellets im Hüttlin Kugelcoater bei einer Zulufttemperatur von 40°C mit 21Gew.-% Eudragit L-55, bezogen auf das Gesamtgewicht der Pellets der nachfolgenden Zusammensetzung aus wäßriger Dispersion überzogen.

| Filmüberzug für 5 kg Pellets: | |
|---|---|
| Eudragit L30D-55 (Röhm, 30% ige, wäßrige Dispersion von Polymethacrylsäureethylacrylat 1:1 Copolymer) | 3500 g |
| Eudragit FS 30D (Röhm, 30% ige, wäßrige Dispersion von Polymethacrylsäuremethylacrylatmethylmethacrylat Copolymer) | 350 g |
| Triethylcitrat | 210 g |
| Glycerinmonostearat (Cutina GMS, Henkel) | 92,4 g |
| Wasser | 3134,6 g |

Anschließend wurden jeweils 322,5 mg Pellets, entsprechend einer Dosis von 75 mg Tramadolhydrochlorid und 50 mg Diclofenac-Natrium, zunächst mit 22,5 mg quervernetztem Polyvinylpyrrolidon (Kollidon CL, BASF) und dann mit 205,6 mg Cellactose (Meggle), 25 mg Tramadolhydrochlorid sowie 1,4 mg Magnesiumstearat gemischt und zu 7 x 14 mm Oblongtabletten mit Bruchkerbe und einem Gewicht von 577 mg verpreßt. Diese zerfallen in wäßrigem Medium wieder in die einzelnen Pellets.

Das Freisetzungsprofil, wie vorstehend angegeben bestimmt, war wie folgt:

| Zeit in min | Freigesetzte Menge in mg pro Tablette für Tramadol Dosis (100 mg) | für Diclofenac Dosis (50 mg) |
|---|---|---|
| 30 | 25 | 0 |
| 120 | 25 | 0 |
| 240 | 65 | 22 |
| 360 | 77 | 31 |
| 420 | 81 | 35 |
| 600 | 91 | 42 |

## Patentansprüche

1. Zumindest teilweise retardierte, orale, pharmazeutische Darreichungsformen des Tramadols, **dadurch gekennzeichnet, daß** deren retardierter Anteil des pharmazeutischen Wirkstoffes durch eine in situ Bildung einer Verbindung aus Tramadol und einem aciden weiteren pharmazeutischen Wirkstoff und/oder Hilfsstoff mit einer Wasserlöslichkeit ≤ 100 mg/ml erreicht wurde.

2. Darreichungsformen nach Anspruch 1, **dadurch gekennzeichnet, daß** die Wasserlöslichkeit ≤ 50 mg/ ml, vorzugsweise ≤ 30 mg/ml, besonders bevorzugt ≤ 10 mg/ml beträgt.

3. Darreichungsformen nach Anspruch 1 oder 2, **dadurch gekennzeichnet, daß** das Tramadol zur Herstellung der in situ gebildeten Verbindung als wasserlösliches Salz, vorzugsweise als Tramadolhydrochlorid, eingesetzt wurde.

4. Darreichungsformen nach einem oder mehreren der Ansprüche 1 bis 3, **dadurch gekennzeichnet, daß** zur Herstellung der in situ gebildeten Verbindung ein wasserlösliches, pharmazeutisch verträgliches Salz eines aciden, weiteren pharmazeutischen Wirkstoffes oder Hilfsstoffes eingesetzt wurde.

5. Darreichungsformen nach Anspruch 4, **dadurch gekennzeichnet, daß** als Salz das Natriumsalz von Diclofenac, Naproxen, Acetylsalicylsäure, der Salicylsäure, der Benzoesäure, des Saccharins, Cyclamats oder Acesulfams eingesetzt wurde.

6. Darreichungsformen nach einem oder mehreren der Ansprüche 1 bis 5, **dadurch gekennzeichnet, daß** die Tramadolkomponente im Überschuß vorliegt.

7. Darreichungsformen nach Anspruch 6, **dadurch gekennzeichnet, daß** das Tramadol mit wenigstens zwei unterschiedlichen Geschwindigkeiten freigesetzt wird.

8. Darreichungsformen nach einem der Ansprüche 1 bis 5, **dadurch gekennzeichnet, daß** das Tramadol und der acide pharmazeutische Wirkstoff oder Hilfsstoff in äquimolaren Mengen als eine in situ gebildete Verbindung vorliegen.

9. Darreichungsfromen nach Anspruch 8, **dadurch gekennzeichnet, daß** das Tramadol und der acide Wirkstoff oder Hilfsstoff mit derselben Geschwindigkeit freigesetzt werden.

10. Darreichungsformen nach einem oder mehreren der Ansprüche 4 bis 9, **dadurch gekennzeichnet, daß** als Wirkstoffe Tramadolhydrochlorid und Diclofenac-Natrium zur in situ Bildung eingesetzt wurden.

11. Darreichungsformen nach Anspruch 10, **dadurch gekennzeichnet, daß** das molare Verhältnis von Tramadol zu Diclofenac 0,5:1 bis 4:1, vorzugsweise 1:1 bis 2:1 beträgt.

12. Darreichungsformen nach Anspruch 10 oder 11, **dadurch gekennzeichnet, daß** Tramadol und Diclofenac zumindest teilweise mit derselben Geschwindigkeit freigesetzt werden.

13. Darreichungsformen nach Anspruch 10, **dadurch gekennzeichnet, daß** Tramadol und Diclofenac äquimolar als eine in situ gebildete Verbindung vorliegen und jeweils die Gesamtmenge jedes Wirkstoffes mit derselben Geschwindigkeit freigesetzt wird .

14. Darreichungsformen nach einem oder mehreren der Ansprüche 1 bis 13, **dadurch gekennzeichnet, daß** sie multipartikulär, vorzugsweise als Granulate, Mikropartikel, Mikrotabletten oder Pellets, bevorzugt als Pellets, gegebenenfalls in Kapseln abgefüllt, formuliert vorliegen.

15. Darreichungsformen nach einem oder mehreren der Ansprüche 1 bis 13, **dadurch gekennzeichnet, daß** sie als Dragees oder Tabletten, vorzugsweise schnell zerfallende Tabletten formuliert vorliegen.

16. Darreichungsformen nach Anspruch 15, **dadurch gekennzeichnet, daß** die Tabletten aus verpreßten Pellets bestehen.

17. Darreichungsformen nach einem oder mehreren der Ansprüche 1 bis 16, **dadurch gekennzeichnet, daß** sie wenigstens einen magensaftresistenten Überzug aufweisen.

18. Darreichungsformen nach einem oder mehreren der Ansprüche 1 bis 17 zur Bekämpfung von Schmerzen.

19. Darreichungsformen nach einem oder mehreren der Ansprüche 1 bis 17 zur Behandlung von Harninkontinenz.

20. Verfahren zur Herstellung zumindest teilweise retardierter, oraler Darreichungsformen nach einem der Ansprüche 1 bis 19, **dadurch gekennzeichnet, daß** Tramadol und ein acider pharmazeutischer Wirkstoff und/oder Hilfsstoff bzw. deren jeweiligen wasserlöslichen Salze und ggf. weitere Hilfsstoffe gemischt, mehrmals angefeuchtet und unter Energieeintrag formuliert werden.

21. Verfahren nach Anspruch 20, **dadurch** gekennzeichet, daß die Mischung mit wässrigen Medien, vorzugsweise Wasser oder wässrigen Bindemittellösungen angefeuchtet wird.

22. Verfahren nach Anspruch 20 oder 21, **dadurch gekennzeichnet, daß** der Energieeintrag in Form von Druck und/oder Wärme erfolgt.

23. Verfahren nach einem der Ansprüche 20 bis 22, **dadurch gekennzeichnet, daß** die Mischung einem mehrmaligem Anfeuchten und Granulieren und mindestens einer einmaligen Extrusion unterworfen und ggf. dann endformuliert wird.

24. Verfahren nach einem der Ansprüche 20 bis 22, **dadurch gekennzeichnet, daß** die Mischung nach mehrmaligem Anfeuchten, Granulieren, Extrudieren und/oder Trocknen ggf. nach Abmischen mit weiteren Hilfsstoffen pelletisiert wird.

25. Verfahren nach einem der Ansprüche 20 bis 23, **dadurch gekennzeichnet, daß** die angefeuchtete Mischung granuliert, extrudiert, erneut angefeuchtet und granuliert, extrudiert und anschließend pelletisiert wird.

26. Verfahren nach einem der Ansprüche 20 bis 23, **dadurch gekennzeichnet, daß** die angefeuchtete Mischung granuliert, getrocknet, erneut angefeuchtet und granuliert, extrudiert und anschließend pelletisiert wird.

27. Verfahren nach Ansprüchen 20 bis 26, **dadurch gekennzeichnet, daß** die Pellets zu Tabletten verpreßt werden.

28. Verfahren nach Anspruch 27, **dadurch gekennzeichnet, daß** die Pellets vor dem Verpressen mit wenigstens einem magensaftresistenten Überzug überzogen werden.

29. Verfahren nach einem oder mehreren der Ansprüche 20 bis 28 , **dadurch gekennzeichnet, daß** als Tramadolsalz Tramadolhydrochlorid und als weiterer Wirkstoff Diclofenac-Natrium eingesetzt werden.

## Claims

1. At least partially sustained-release, oral pharmaceutical forms of administration of tramadol, **characterized in that** the sustained-release portion of the pharmaceutical active substance is achieved by in situ formation of a compound of tramadol and another, acidic pharmaceutical active substance and/or auxiliary substance with a water solubility of ≤100 mg/ml.

2. Forms of administration according to Claim 1, **characterized in that** the water solubility is ≤50 mg/ml, preferably ≤30 mg/ml and particularly preferably ≤10 mg/ml.

3. Forms of administration according to Claim 1 or 2, **characterized in that** the tramadol has been used as a water-soluble salt, preferably as tramadol hydrochloride, to prepare the compound formed in situ.

4. Forms of administration according to one or more of Claims 1 to 3, **characterized in that** a water-soluble, pharmaceutically acceptable salt of another, acidic pharmaceutical active substance or auxiliary substance has been used to prepare the compound formed in situ.

5. Forms of administration according to Claim 4, **characterized in that** the salt which has been used is the sodium salt of diclofenac, naproxen, acetylsalicylic acid, salicylic acid, benzoic acid, saccharin, cyclamate or acesulfame.

6. Forms of administration according to one or more of Claims 1 to 5, **characterized in that** the tramadol component is present in excess.

7. Forms of administration according to Claim 6, **characterized in that** the tramadol is released at two or more different rates.

8. Forms of administration according to one of Claims 1 to 5, **characterized in that** the tramadol and the acidic pharmaceutical active substance or auxiliary substance are present in equimolar amounts as a compound formed in situ.

9. Forms of administration according to Claim 8, **characterized in that** the tramadol and the acidic active substance or auxiliary substance are released at the same rate.

10. Forms of administration according to one or more of Claims 4 to 9, **characterized in that** tramadol hydrochloride and diclofenac sodium have been used as the active substances for the in situ formation.

11. Forms of administration according to Claim 10, **characterized in that** the molar ratio of tramadol to diclofenac is 0.5:1 to 4:1 and preferably 1:1 to 2:1.

12. Forms of administration according to Claim 10 or 11, **characterized in that** at least part of the tramadol and at least part of the diclofenac are released at the same rate.

13. Forms of administration according to Claim 10, **characterized in that** the tramadol and the diclofenac are present in equimolar amounts as a compound formed in situ and the total amount of each active substance is released at the same rate.

14. Forms of administration according to one or more of Claims 1 to 13, **characterized in that** they are multiparticulate formulations, preferably in the form of granules, microparticles, microtablets or pellets and particularly preferably in the form of pellets, optionally filled into capsules.

15. Forms of administration according to one or more of Claims 1 to 13, **characterized in that** they are formulated as coated tablets or ordinary tablets, preferably as rapidly disintegrating tablets.

16. Forms of administration according to Claim 15, **characterized in that** the tablets consist of compressed pellets.

17. Forms of administration according to one or more of Claims 1 to 16, **characterized in that** they have at least one enteric coating.

18. Forms of administration according to one or more of Claims 1 to 17 for the control of pain.

19. Forms of administration according to one or more of Claims 1 to 17 for the treatment of urinary incontinence.

20. Process for the preparation of at least partially sustained-release, oral forms of administration according to one of Claims 1 to 19, **characterized in that** tramadol and an acidic pharmaceutical active substance and/or auxiliary substance or water-soluble salts thereof, and optionally other auxiliary substances, are mixed, moistened several times and formulated under an energy input.

21. Process according to Claim 20, **characterized in that** the mixture is moistened with aqueous media, preferably water or aqueous binder solutions.

22. Process according to Claim 20 or 21, **characterized in that** the energy input takes the form of pressure and/or heat.

23. Process according to one of Claims 20 to 22, **characterized in that** the mixture is moistened and granulated several times, extruded at least once and then optionally converted to the final formulation.

24. Process according to one of Claims 20 to 22, **characterized in that**, after all these moistening, granulation, extrusion and/or drying steps, the mixture is optionally mixed with other auxiliary substances and then pelleted.

25. Process according to one of Claims 20 to 23, **characterized in that** the moistened mixture is granulated, extruded, moistened and granulated again, extruded and then pelleted.

26. Process according to one of Claims 20 to 23, **characterized in that** the moistened mixture is granulated, dried, moistened and granulated again, extruded and then pelleted.

27. Process according to Claims 20 to 26, **characterized in that** the pellets are compressed to tablets.

28. Process according to Claim 27, **characterized in that** the pellets are provided with at least one enteric coating before being compressed.

29. Process according to one or more of Claims 20 to 28, **characterized in that** tramadol hydrochloride is used as the tramadol salt and diclofenac sodium is used as the other active substance.

## Revendications

1. Formes pharmaceutiques du tramadol administrables par voie orale, à action au moins partiellement retardée, **caractérisées en ce que** leur fraction retardée de la substance pharmaceutique active a été obtenue par formation in situ d'un composé du tramadol et d'une autre substance pharmaceutique active acide et/ou d'une substance auxiliaire acide, de solubilité dans l'eau inférieure ou égale à 100 mg/ml.

2. Formes d'administration suivant la revendication 1, **caractérisées en ce que** la solubilité dans l'eau est inférieure ou égale à 50 mg/ml, avantageusement inférieure ou égale à 30 mg/ml, notamment inférieure ou égale à 10 mg/ml.

3. Formes d'administration suivant la revendication 1 ou 2, **caractérisées en ce que** le tramadol a été utilisé pour la préparation du composé formé in situ comme sel hydrosoluble, avantageusement comme chlorhydrate de tramadol.

4. Formes d'administration suivant l'une ou plusieurs des revendications 1 à 3, **caractérisées en ce qu'**un sel hydrosoluble acceptable du point de vue pharmaceutique .d'une autre substance pharmaceutique active acide ou substance auxiliaire acide a été utilisé pour la préparation du composé formé in situ.

5. Formes d'administration suivant la revendication 4, **caractérisées en ce que** le sel qui a été utilisé est le sel de sodium du diclofénac, du naproxène, de l'acide acétylsalicylique, de l'acide salicylique, de l'acide benzoique, de la saccharine, du cyclamate ou de l'acésulfam.

6. Formes d'administration suivant l'une ou plusieurs des revendications 1 à 5, **caractérisées en ce que** le composant tramadol est présent en excès.

7. Formes d'administration suivant la revendication 6, **caractérisées en ce que** le tramadol est libéré à au moins deux vitesses différentes.

8. Formes d'administration suivant l'une des revendications 1 à 5, **caractérisées en ce que** le tramadol et la substance pharmaceutique active acide ou la substance auxiliaire acide sont présents en quantité équimolaire sous forme d'un composé formé in situ.

9. Formes d'administration suivant la revendication 8, **caractérisées en ce que** le tramadol et la substance active acide ou substance auxiliaire acide sont libérés à la même vitesse.

10. Formes d'administration suivant l'une ou plusieurs des revendications 4 à 9, **caractérisées en ce que** le chlorhydrate de tramadol et le sel de sodium du diclofénac ont été utilisés comme substances actives pour la formation in situ.

11. Formes d'administration suivant la revendication 10, **caractérisées en ce que** le rapport molaire du tramadol au diclofénac est de 0,5:1 à 4:1, avantageusement de 1:1 à 2:1.

12. Formes d'administration suivant la revendication 10 ou 11, **caractérisées en ce que** le tramadol et le diclofénac sont libérés au moins partiellement à la même vitesse.

13. Formes d'administration suivant la revendication 10, **caractérisées en ce que** le tramadol et le diclofénac sont présents en proportion équimolaire sous forme d'un composé formé in situ et la quantité totale de chaque substance active est libérée dans chaque cas à la même vitesse.

14. Formes d'administration suivant l'une ou plusieurs des revendications 1 à 13, **caractérisées en ce qu'**elles ont une formulation multiparticulaire, avantageusement sous forme de granulés, microparticules, microtablettes ou pellets, avantageusement sous forme de pellets éventuellement chargés dans des gélules.

15. Formes d'administration suivant l'une ou plusieurs des revendications 1 à 13, **caractérisées en ce qu'**elles sont formulées en dragées ou comprimés, avantageusement en comprimés à désintégration rapide.

16. Formes d'administration suivant la revendication 15, **caractérisées en ce que** les comprimés sont constitués de pellets pressés.

17. Formes d'administration suivant l'une ou plusieurs des revendications 1 à 16, **caractérisées en ce qu'**elles présentent au moins un revêtement résistant au suc gastrique.

18. Formes d'administration suivant l'une ou plusieurs des revendications 1 à 17, destinées à combattre des douleurs.

19. Formes d'administration suivant l'une ou plusieurs des revendications 1 à 17, destinées au traitement de l'incontinence urinaire.

20. Procédé de production de formes d'administration orales au moins partiellement retardées suivant l'une ou plusieurs des revendications 1 à 19, **caractérisé en ce qu'**on mélange du tramadol et une substance pharmaceutique active et/ou une substance auxiliaire acides ou leurs sels hydrosolubles respectifs et, le cas échéant, d'autres substances auxiliaires, on humecte plusieurs fois le mélange et on le formule avec apport d'énergie.

21. Procédé suivant la revendication 20, **caractérisé en ce que** le mélange est humecté avec des milieux aqueux, avantageusement de l'eau ou des solutions aqueuses d'un liant.

22. Procédé suivant la revendication 20 ou 21, **caractérisé en ce que** l'apport d'énergie est effectué sous forme de pression et/ou de chaleur.

23. Procédé suivant l'une des revendications 20 à 22, **caractérisé en ce que** le mélange est soumis à une opération répétée d'humectation et de granulation et à une extrusion effectuée au moins une fois, puis le cas échéant transformé en formulation finale.

24. Procédé suivant l'une des revendications 20 à 22, **caractérisé en ce que** le mélange est transformé en pellets après une opération répétée d'humectation, de granulation, d'extrusion et/ou de séchage, éventuellement après adjonction d'autres substances auxiliaires.

25. Procédé suivant l'une des revendications 20 à 23, **caractérisé en ce que** le mélange humecté est granulé, extrudé, de nouveau humecté et granulé, extrudé puis transformé en pellets.

26. Procédé suivant l'une des revendications 20 à 23, **caractérisé en ce que** le mélange humecté est granulé, séché, de nouveau humecté et granulé, extrudé puis transformé en pellets.

27. Procédé suivant les revendications 20 à 26, **caractérisé en ce que** les pellets sont pressés en comprimés.

28. Procédé suivant la revendication 27, **caractérisé en ce que** les pellets, avant la compression, sont revêtus d'au moins un enrobage résistant au suc gastrique.

29. Procédé suivant l'une ou plusieurs des revendications 20 à 28, **caractérisé en ce qu'**on utilise comme sel de tramadol le chlorhydrate de tramadol et comme autre substance active, le sel de sodium du diclofénac.
